# EUROPEAN PATENT SPECIFICATION

(11) **EP 4 091 794 B1**
(45) Date of publication and mention of the grant of the patent: **30.04.2025**
(21) Application number: 20913539.1
(22) Date of filing: 30.07.2020
(51) Int. Cl.: B29C 49/42, A61L 2/22, A61L 2/04, B29C 49/64, B29L 31/00

(54) **PREFORM DISINFECTION METHOD AND BOTTLE MANUFACTURING DEVICE**
VERFAHREN ZUR DESINFEKTION VON VORFORMEN UND VORRICHTUNG ZUR HERSTELLUNG VON FLASCHEN
PROCÉDÉ DE DÉSINFECTION DE PRÉFORME ET DISPOSITIF DE FABRICATION DE BOUTEILLE

(30) Priority: 19.01.2020 CN 202010058120
(43) Date of publication of application: 23.11.2022
(73) Proprietor: Jiangsu Newamstar Packaging Machinery Co., Ltd, Suzhou, Jiangsu 215618 (CN)
(72) Inventor: YE, Peng, Suzhou, Jiangsu 215618 (CN)
(74) Representative: Fidal Innovation
(86) International application number: PCT/CN2020/105739
(87) International publication number: WO 2021/143099

(56) References cited:
- EP-B1- 2 623 293
- CN-A- 1 264 332
- CN-A- 1 264 332
- CN-A- 101 203 370
- CN-A- 101 203 370
- CN-A- 102 711 845
- CN-A- 102 711 845
- CN-A- 102 711 845
- CN-A- 105 682 886
- CN-A- 105 682 886
- CN-A- 111 231 273
- CN-U- 215 775 323
- ES-T3- 2 374 985
- JP-A- 2015 013 482
- KR-A- 20180 119 895
- PT-E- 2 094 312
- US-B2- 9 050 381

## Description

### Technical Field of the Invention

The present disclosure relates to the field of bottle-blowing techniques, in particular to a preform disinfection method and a bottle manufacturing device.

### Background of the Invention

As the beverage industry has higher and higher requirements for food safety, more and more attention is paid to its pollution during the beverage packaging process. Especially for dairy beverages, contamination of packaging utensils will directly affect the quality of the product. At present, most of the industry uses disinfectant to sterilize utensils and then wash them, but there is a large consumption of disinfectant and sterile water, and there are even residual problems, which are likely to cause secondary pollution.

At present, there are methods and devices for blowing a bottle after spraying a disinfectant on a preform, then heating and sterilizing it. In such a device, high temperature disinfectant vapor is sprayed to make it condense on the surface of the preform. With this method, it is not only necessary to keep the preform at a low initial temperature (lower than the condensation temperature of the disinfectant vapor), but also to preheat the liquid disinfectant to prepare the disinfectant vapor, which consumes a lot of energy and has a relatively high preparation cost of the equipment; at the same time, due to the high temperature of the disinfectant vapor, the disinfectant is partially decomposed in the vapor state, which affects its disinfection effect.

US9050381B2 provided a method and a device for sterilizing the surface of a space-to-be-sterilized which are safe to the human body, which are effective even for sterilizing spore-forming bacteria, which do not require the space-to-be-sterilized to be highly shielded, and which can sterilize the surface of the space efficiently with inexpensive equipment. The disclosed method involves: spraying a low-concentration sterilizing agent in the form of fine particles into the space-to-be-sterilized and making the sterilizing agent adhere to the surface thereof, the low-concentration sterilizing agent being any one of a 1-10 wt % aqueous solution of hydrogen peroxide, a 0.1-1 wt % aqueous solution of peracetic acid, or a 0.01-0.1 wt % aqueous solution of hypochlorous acid; adjusting the humidity in the space-to-be-sterilized; and air-drying the sterilizing agent. Thus, a high level of sterilization can be achieved in a short time and at low cost.

PT2094312E involves preheating a preform (12) by a heating unit (32) e.g. infrared lamp, till a set point temperature (TC1). The preform is exposed to UV emitted by a sterilizing unit (76) during a determined time lag. The preform is heated by another heating unit (34) till another set point temperature so as to sterilize an external surface (28) of a tubular body (14) of the preform till determined decontamination degree. The sterilizing unit is formed of UV lamp (78) and reflectors (80). Independent claims are also included for the following: (1) an installation for fabricating sterile containers (2) a furnace for thermal conditioning of a preform.

### Summary of the Invention

The first object of the present disclosure is to provide a preform disinfection method, in which the disinfectant solution is directly sprayed after ultrasonic atomization, so that it is uniformly attached to the surfaces of the preforms. There is no need to heat the disinfectant solution to the vaporization temperature, allowing low energy consumption, and avoiding the loss caused by making the disinfectant solution into disinfectant solution vapor.

To achieve the above purpose, the technical solution employed by the present disclosure is:
A preform disinfection method, the preforms being disinfected and molded in a device for producing sterile bottles, comprises at least the following step:
   (1) making a disinfectant solution into disinfectant solution aerosol by means of an ultrasonic atomizer, and spraying the disinfectant solution aerosol onto the preforms, so as to attach a substantially uniform thin layer of disinfectant solution to at least the inner surfaces of the preforms to be disinfected;
   (2) heating the preforms treated in step (1) by radiation to raise a temperature of at least the inner surfaces of the preforms to a first temperature, where the first temperature is greater than or equal to the activation temperature of the disinfectant solution, so as to disinfect at least the inner surfaces of the preforms;
in step (1), first heating the disinfectant solution or the disinfectant solution aerosol to raise a temperature of the disinfectant solution aerosol to a second temperature, then spraying the disinfectant solution aerosol raised to the second temperature onto the preforms, where the second temperature is greater than the temperature of the preforms before heating in step (2) and lower than the first temperature.

More preferably, the disinfection method further comprises an auxiliary disinfection step:
(3) after step (2), spraying the disinfectant solution aerosol onto the preforms heated to the first temperature again.

More preferably, the first temperature is less than or equal to the blow molding temperature of the preforms and greater than or equal to the vaporization temperature of the disinfectant solution, so that the disinfectant solution evaporates from the preforms.

The second object of the present disclosure is to provide a bottle manufacturing device, which adopts the above-mentioned preform disinfection method, in which the disinfectant solution is directly sprayed after ultrasonic atomization, so that it is uniformly attached to the surfaces of the preforms. There is no need to heat the disinfectant solution to the vaporization temperature, allowing low energy consumption, and avoiding the loss caused by making the disinfectant solution into disinfectant solution vapor.

To achieve the above purpose, the technical solution employed by the present disclosure is:
A bottle manufacturing device for producing sterile bottles from plastic preforms, the preforms being continuously conveyed to the bottle manufacturing device, comprises the following components successively arranged along the conveying direction of the preforms:
a spraying unit; the spraying unit comprises a sprayer for spraying a disinfectant medium to at least the inner surfaces of the preforms;
a heating unit; the heating unit comprises a heating furnace for radiation heating the preforms to a first temperature which is less than or equal to the blow molding temperature of the preforms and is greater than or equal to the activation temperature of a disinfectant solution aerosol;
a molding unit; the molding unit is used to blow the preforms to manufacture sterile bottles;
the spraying unit further comprises an ultrasonic atomizer for making a disinfectant solution into disinfectant solution aerosol, and the sprayer is to spray the disinfectant solution aerosol onto the preforms to attach a substantially uniform thin layer of disinfectant solution to at least the inner surfaces of the preforms to be disinfected;
the heating unit is to heat the preforms after being sprayed by the spraying unit, so as to disinfect at least the inner surfaces of the preforms;
the molding unit is to blow mold the preforms having the blow molding temperature heated by the heating unit to manufacture sterile bottles;
the spraying unit further comprises a heater for heating the disinfectant solution or the disinfectant solution aerosol to raise a temperature of the disinfectant solution aerosol to a second temperature which is greater than the temperature of the preforms before heating in the heating unit and lower than the first temperature, and the sprayer is to spray the disinfectant solution aerosol with the second temperature onto the preforms, to attach a substantially uniform thin layer of disinfectant solution to at least the inner surfaces of the preforms to be disinfected.

Preferably, the heating unit is to heat the preforms after being sprayed by the spraying unit to the first temperature which is greater than or equal to the vaporization temperature of the disinfectant solution aerosol, so as to evaporate the disinfectant solution from the preforms.

Preferably, the disinfectant solution is a solution containing hydrogen peroxide, which is to be activated after the preforms are heated to a temperature exceeding the activation temperature.

Due to the use of the above technical solutions, the present disclosure has the following advantages over the prior art: In a preform disinfection method and a bottle manufacturing device of the present disclosure, the disinfectant solution is directly sprayed after ultrasonic atomization, so that it is uniformly attached to the surfaces of the preforms. There is no need to heat the disinfectant solution to the vaporization temperature, allowing low energy consumption, and avoiding the loss caused by making the disinfectant solution into disinfectant solution vapor. This method can not only avoid the problem of insufficient gasification that affects the bottle molding quality due to excessive accumulation of disinfectant solution on the surfaces of the preforms, but also saves the consumption of disinfectant solution to a large extent and saves costs.

### Brief Description of the Drawings

Figure 1 is a first schematic structure diagram of the first embodiment of the device for manufacturing sterile bottles by applying the method of the present disclosure;
Figure 2 is a second schematic structure diagram of the first embodiment of the device for manufacturing sterile bottles by applying the method of the present disclosure;
Figure 3 is a first schematic structure diagram of the second embodiment of the device for manufacturing sterile bottles by applying the method of the present disclosure;
Figure 4 is a second schematic structure diagram of the second embodiment of the device for manufacturing sterile bottles by applying the method of the present disclosure;
Figure 5 is a schematic structure diagram of the spraying unit;

Wherein, 1 - preform; 2 - spraying unit; 3 - heating unit; 4 - molding unit; 5 - bottle; 6 - ultrasonic atomizer; 7 - sprayer; 8 - first conveying mechanism; 9 - second conveying mechanism.

### Detailed Description of Exemplary Embodiments

In the following, the technical solution of the present disclosure is further described combining with the accompanying drawings.

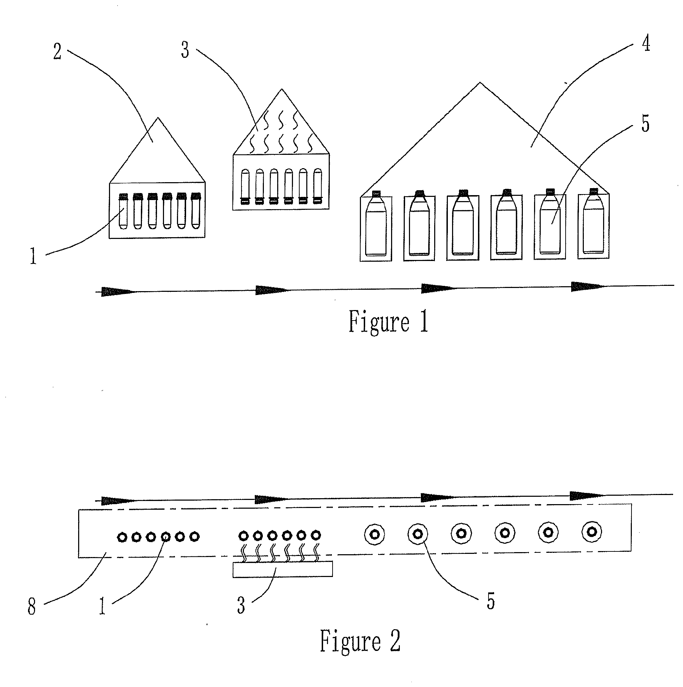

Referring to Figures 1 - 5, the above-mentioned bottle manufacturing device is to produce sterile bottles 5 through plastic preforms 1, and the preforms 1 are continuously conveyed to the bottle manufacturing device. The bottle manufacturing device comprises a spraying unit 2, a heating unit 3 and a molding unit 4 through which the preforms 1 are transferred.

The preforms 1 are tubular as a whole, one end of each tubular preform 1 is closed, and the other end is the same shape as the neck of the bottles 5 after molding. Since the bottle manufacturing device is used to manufacture sterile bottles 5, the preforms 1 need to be disinfected and sterilized and then blown, so that the preforms 1 have a certain degree of sterility. The disinfection and sterilization step is used to disinfected and sterilize at least the inner surfaces of the preforms 1 (the inner surfaces include the inner walls and the inner sides of the necks).

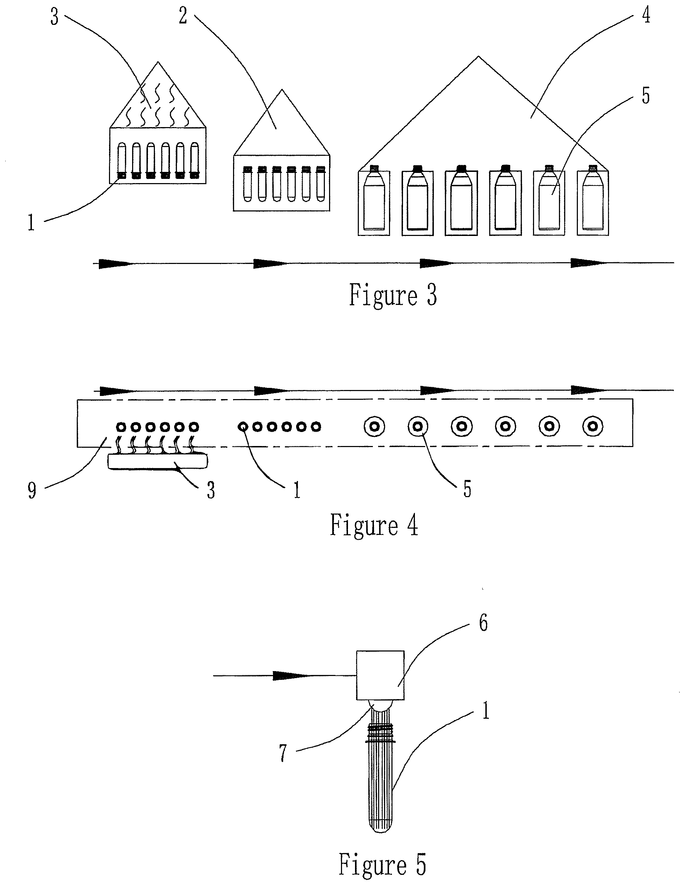

Referring to Figure 5, the spraying unit 2 comprises an ultrasonic atomizer 6 for ultrasonically vibrating and atomizing the disinfectant to form a disinfectant solution aerosol, and a sprayer 7 for spraying the disinfectant solution aerosol to uniformly attach it to at least the inner surfaces of the preforms 1. And the outlet of the ultrasonic atomizer 6 communicates with the inlet of the sprayer 7. The ultrasonic atomizer 6 utilizes the principle of ultrasonic oscillating atomization to generate disinfectant solution aerosol, which is sprayed into the preforms 1 through the sprayer 7 to cover at least the inner surfaces of the preforms 1, so as to attach a substantially uniform thin layer of disinfectant solution on at least the inner surfaces of the preforms 1 to be disinfected, in this embodiment, the disinfectant solution is a solution containing hydrogen peroxide, which is to be activated after the preforms 1 are heated to a temperature exceeding the activation temperature. The disinfectant solution is activated after reaching or exceeding the activation temperature, having the best disinfection effect. Specifically, the hydrogen peroxide is activated after reaching or exceeding the activation temperature, and rapidly decomposes to produce new oxygen, which destroys the protein on the surface of the microorganisms through oxidation, thereby killing the microorganisms.

In this embodiment, the spraying unit 2 further comprises a heater (not shown in the figures) for heating the disinfectant solution or disinfectant solution aerosol to raise a temperature of the disinfectant solution aerosol to a second temperature, which is slightly higher than the temperature of the preforms 1. The heater can be to first heat the disinfectant solution to the second temperature, and then the disinfectant solution with the second temperature passes through the ultrasonic atomizer 6 to prepare the disinfectant solution aerosol with the second temperature; the heater can also directly heat the disinfectant solution aerosol produced by the ultrasonic atomizer 6 to make it have the second temperature.

The sprayer 7 is to spray the disinfectant solution aerosol with the second temperature so as to uniformly attach it onto at least the inner surfaces of the preforms 1, so as to attach a substantially uniform thin layer of disinfectant solution on at least the inner surfaces of the preforms 1 to be disinfected. Through this configuration, a temperature difference is generated between the disinfectant solution aerosol and the preforms 1, and the attachment effect of the disinfectant solution aerosol is improved. The heater is only used to raise a temperature of the disinfectant solution aerosol to a second temperature greater than the temperature of the preforms 1, and the energy consumption is relatively low. The second temperature is much lower than the first temperature.

The heating unit 3 comprises a heating furnace for radiation heating the preforms 1 to a first temperature, the first temperature is less than or equal to the blow molding temperature of the preforms 1. In this embodiment, the first temperature is equal to the blow molding temperature. Through this configuration, when the preforms 1 with the first temperature reaches the blow molding temperature (namely the rubbery state temperature), they can be blow molded directly in the molding unit 4 without heating the preforms 1 again before blow molding.

The first temperature is greater than or equal to the activation temperature of the disinfectant solution aerosol. Through this configuration, when the preforms 1 enter the heating unit 3 and are heated to the first temperature, the disinfectant solution aerosol is activated to disinfect and sterilize at least the inner surfaces of the preforms 1.

The first temperature is greater than or equal to the vaporization temperature of the disinfectant solution aerosol. Through this configuration, when the preforms 1 enter the heating unit 3 and are heated to the first temperature, the disinfectant solution aerosol can be evaporated from the surfaces of the preforms 1, avoiding the disinfectant solution aerosol remaining on the surfaces of the preforms 1 and affecting the molding quality of the bottles 5.

The molding unit 4 comprises a blow molding station for blowing the preforms 1 to produce bottles 5, and the blow molding station is to blow mold the preforms 1 in a mold cavity. The blow molding station should comprise a mold cavity for loading the preforms 1, for blowing sterile compressed air into the preforms 1 so that the inside of the preforms 1 is compressed and expanded to the shape of the mold cavity, so as to produce sterile bottles 5.

Figures 1 - 2 are schematic structure diagrams of the first embodiment of the device for manufacturing sterile bottles by applying the method of the present disclosure, the bottle manufacturing device further comprises a first conveying mechanism 8, and the first conveying mechanism 1 is to convey the preforms 1 to pass through the spraying unit 2, the heating unit 3 and the molding unit 4 successively. The first conveying mechanism 8 may be composed of a plurality of conveying star wheels. The first conveying mechanism 8 feeds the preforms 1 with a thin layer of disinfectant solution substantially uniformly attached to at least the inner surfaces thereof to the heating unit 3, the heating unit 3 heats the preforms 1 to the first temperature, the disinfectant solution is used for disinfecting and sterilizing at least the inner surfaces of the preforms 1, and the residual disinfectant solution is evaporated, and the first conveying mechanism 8 then sends the sterilized preforms 1 with the first temperature to the molding unit 4 for blow molding.

Since the blow molding temperature, the vaporization temperature and the activation temperature are relatively close, when the preforms 1 are conveyed in the heating furnace, the activation and vaporization of the disinfectant solution are gradually realized in the process that the temperature gradually rises to the first temperature.

Figures 3 - 4 are schematic structure diagrams of the second embodiment of the device for manufacturing sterile bottles by applying the method of the present disclosure, the bottle manufacturing device further comprises a second conveying mechanism 9, and the second conveying mechanism 1 is to convey the preforms 1 to pass through the heating unit 3, the spraying unit 2 and the molding unit 4 successively. The second conveying mechanism 9 may be composed of a plurality of conveying star wheels. The second conveying mechanism 9 feeds the preforms 1 with the first temperature into the spraying unit 2, the spraying unit 2 sprays disinfectant solution aerosol to the preforms 1, the disinfectant solution disinfects and sterilizes at least the inner surfaces of the preforms 1, the residual disinfectant solution is evaporated, and the second conveying mechanism 9 then sends the sterilized preforms 1 to the molding unit 4 for blow molding. The first temperature is less than or equal to the blow molding temperature, and since the blow molding temperature is the rubbery state temperature of the preforms 1, the preforms 1 will not deform at the rubbery state temperature, and heating the preforms 1 first will not affect the disinfecting effect of the disinfectant solution aerosol. When the preforms 1 pass through the spraying unit 2 and the temperature drops below the blow molding temperature, the preforms 1 are heated again to the blow molding temperature, and then the preforms 1 are sent to the molding unit 4.

The following specifically describes the working process of this embodiment:
The disinfection method for disinfecting the plastic preforms 1 used for molding in the device for producing sterile bottles 5 comprises at least the following steps:
(1) making a disinfectant solution into disinfectant solution aerosol by means of an ultrasonic atomizer 6, and spraying the disinfectant solution aerosol onto the preforms 1, so as to attach a substantially uniform thin layer of disinfectant solution to at least the inner surfaces of the preforms 1 to be disinfected; according to needs, a thin layer of disinfectant solution may also be attached onto the outer surfaces of the preforms 1.
(2) radiation heating the preforms 1 thus treated to raise a temperature of at least the inner surfaces of the preforms 1 to a first temperature, to at least further disinfect the inner surfaces of the preforms 1, where the first temperature is greater than or equal to the activation temperature of the disinfectant solution, and when the outer surfaces of the preforms 1 are also attached with a thin layer of disinfectant solution, the outer surfaces of the preforms 1 can be disinfected at the same time by radiant heating.

The disinfection method further comprises an auxiliary disinfection step:
(3) after step (2), spraying the disinfectant solution aerosol onto the preforms 1 heated to the first temperature, to further disinfect the preforms 1.

In this embodiment, the first temperature is equal to the blow molding temperature of the preforms 1 and greater than or equal to the vaporization temperature of the disinfectant solution, so that the disinfectant solution evaporates from the preforms 1; at the same time, the preforms 1 with the first temperature meet the blow molding conditions.

It is also possible to first heat the preforms 1 by radiation to raise a temperature of at least the inner surfaces of the preforms 1 to the first temperature, and then spray the disinfectant solution aerosol formed by ultrasonic vibration and atomization to the preforms 1 to disinfect and sterilize at least the inner surfaces of the preforms 1. According to needs, the disinfectant solution aerosol can also be sprayed to the outer surfaces of the preforms 1 at the same time, so as to disinfect and sterilize the inners and outers surfaces of the preforms 1 at the same time.

When step (1) and step (2) are performed in sequence, in step (1), the disinfectant solution or disinfectant solution aerosol can be heated first so that the disinfectant solution aerosol rises to the second temperature, which is greater than that of the preforms 1, then, the disinfectant solution aerosol raised to the second temperature is sprayed to at least the inner surfaces of the preforms 1. The temperature difference generated between the disinfectant solution aerosol and the preforms 1 improves the attachment effect of the disinfectant solution aerosol.

After the preforms 1 are disinfected, they are fed into the molding unit 4, and the preforms 1 are blow molded in the mold cavity by the blow molding station.

In the above-mentioned method, when only the preforms 1 need to be disinfected, the first temperature is less than or equal to the blow molding temperature of the preforms 1. When blow molding is required and the temperature of the preforms 1 are lower than the blow molding temperature, it is only necessary to reheat the preforms 1 to raise their temperature to the blow molding temperature.

## Claims

1. A preform disinfection method, the preforms (1) being disinfected and molded in a device for producing sterile bottles (5), the disinfection method comprises at least the following step:
(1) making a disinfectant solution into disinfectant solution aerosol by means of an ultrasonic atomizer (6), and spraying the disinfectant solution aerosol onto the preforms (1), so as to attach a substantially uniform thin layer of disinfectant solution to at least the inner surfaces of the preforms (1) to be disinfected;
(2) heating the preforms (1) treated in step (1) by radiation to raise a temperature of at least the inner surfaces of the preforms (1) to a first temperature, where the first temperature is greater than or equal to the activation temperature of the disinfectant solution, so as to disinfect at least the inner surfaces of the preforms (1);
in step (1), first heating the disinfectant solution or the disinfectant solution aerosol to raise a temperature of the disinfectant solution aerosol to a second temperature, then spraying the disinfectant solution aerosol raised to the second temperature onto the preforms (1), where the second temperature is greater than the temperature of the preforms (1) before heating in step (2) and lower than the first temperature.

2. The preform disinfection method according to claim 1, is **characterized in that**, the disinfection method further comprises an auxiliary disinfection step:
(3) after step (2), spraying the disinfectant solution aerosol onto the preforms (1) heated to the first temperature again.

3. The preform disinfection method according to claim 1, is **characterized in that**, the first temperature is less than or equal to the blow molding temperature of the preforms (1) and greater than or equal to the vaporization temperature of the disinfectant solution, so that the disinfectant solution evaporates from the preforms (1).

4. A bottle manufacturing device for producing sterile bottles (5) from plastic preforms (1), the preforms (1) being continuously conveyed to the bottle manufacturing device, comprises the following components successively arranged along the conveying direction of the preforms (1):
a spraying unit (2); the spraying unit (2) comprises a sprayer (7) for spraying a disinfectant medium to at least the inner surfaces of the preforms (1);
a heating unit (3); the heating unit (3) comprises a heating furnace for radiation heating the preforms (1) to a first temperature which is less than or equal to the blow molding temperature of the preforms (1) and is greater than or equal to the activation temperature of a disinfectant solution aerosol;
a molding unit (4); the molding unit (4) is used to blow the preforms (1) to manufacture sterile bottles (5);
wherein the spraying unit (2) further comprises an ultrasonic atomizer (6) for making a disinfectant solution into disinfectant solution aerosol, and the sprayer (7) is to spray the disinfectant solution aerosol onto the preforms (1) to attach a substantially uniform thin layer of disinfectant solution to at least the inner surfaces of the preforms (1) to be disinfected;
the heating unit (3) is to heat the preforms (1) after being sprayed by the spraying unit (2), so as to disinfect at least the inner surfaces of the preforms (1);
the molding unit (4) is to blow mold the preforms (1) having the blow molding temperature heated by the heating unit (3) to manufacture sterile bottles (5);
the spraying unit (2) further comprises a heater for heating the disinfectant solution or the disinfectant solution aerosol to raise a temperature of the disinfectant solution aerosol to a second temperature which is greater than the temperature of the preforms (1) before heating in the heating unit (3) and lower than the first temperature, and the sprayer (7) is to spray the disinfectant solution aerosol with the second temperature onto the preforms (1), to attach a substantially uniform thin layer of disinfectant solution to at least the inner surfaces of the preforms (1) to be disinfected.

5. The bottle manufacturing device according to claim 4, is **characterized in that**, the heating unit (3) is to heat the preforms (1) after being sprayed by the spraying unit (2) to the first temperature which is greater than or equal to the vaporization temperature of the disinfectant solution aerosol, so as to evaporate the disinfectant solution from the preforms (1).

6. The bottle manufacturing device according to claim 4, is **characterized in that**, the disinfectant solution is a solution containing hydrogen peroxide, which is to be activated after the preforms (1) are heated to a temperature exceeding the activation temperature.

## Patentansprüche

1. Vorformlingdesinfektionsverfahren, wobei die Vorformlinge (1) desinfiziert und in einer Vorrichtung zur Herstellung steriler Flaschen (5) geformt werden, wobei das Desinfektionsverfahren mindestens den folgenden Schritt beinhaltet:
(1) Umwandeln einer Desinfektionsmittellösung in ein Desinfektionsmittellösungsaerosol mittels eines Ultraschallzerstäubers (6) und Sprühen des Desinfektionsmittellösungsaerosols auf die Vorformlinge (1), um eine im Wesentlichen gleichmäßige dünne Desinfektionsmittellösungsschicht auf mindestens die Innenflächen der zu desinfizierenden Vorformlinge (1) aufzubringen;
(2) Erhitzen der in Schritt (1) behandelten Vorformlinge (1) durch Bestrahlen, um eine Temperatur mindestens der Innenflächen der Vorformlinge (1) auf eine erste Temperatur zu erhöhen, wobei die erste Temperatur gleich der oder höher als die Aktivierungstemperatur der Desinfektionsmittellösung ist, um mindestens die Innenflächen der Vorformlinge (1) zu desinfizieren;
in Schritt (1) zunächst Erhitzen der Desinfektionsmittellösung oder des Desinfektionsmittellösungsaerosols, um eine Temperatur des Desinfektionsmittellösungsaerosols auf eine zweite Temperatur zu erhöhen, dann Sprühen des auf die zweite Temperatur erhöhten Desinfektionsmittellösungsaerosols auf die Vorformlinge (1), wobei die zweite Temperatur höher als die Temperatur der Vorformlinge (1) vor dem Erhitzen in Schritt (2) und niedriger als die erste Temperatur ist.

2. Vorformlingdesinfektionsverfahren nach Anspruch 1, **dadurch gekennzeichnet, dass** das Desinfektionsverfahren außerdem einen zusätzlichen Desinfektionsschritt beinhaltet:
(3) nach Schritt (2) erneutes Sprühen des Desinfektionsmittellösungsaerosols auf die auf die erste Temperatur erhitzten Vorformlinge (1).

3. Vorformlingdesinfektionsverfahren nach Anspruch 1, **dadurch gekennzeichnet, dass** die erste Temperatur gleich der oder niedriger als die Blasformtemperatur der Vorformlinge (1) und gleich der oder höher als die Verdampfungstemperatur der Desinfektionsmittellösung ist, so dass die Desinfektionsmittellösung aus den Vorformlingen (1) verdampft.

4. Flaschenherstellungsvorrichtung zur Herstellung steriler Flaschen (5) aus Kunststoffvorformlingen (1), wobei die Vorformlinge (1) der Flaschenherstellungsvorrichtung kontinuierlich zugeführt werden, die die folgenden, entlang der Förderrichtung der Vorformlinge (1) hintereinander angeordneten Komponenten umfasst:
eine Sprüheinheit (2); wobei die Sprüheinheit (2) einen Sprüher (7) zum Sprühen eines Desinfektionsmittels auf mindestens die Innenflächen der Vorformlinge (1) umfasst;
eine Heizeinheit (3); wobei die Heizeinheit (3) einen Heizofen zur Strahlungserhitzung der Vorformlinge (1) auf eine erste Temperatur umfasst, die gleich der oder niedriger als die Blasformtemperatur der Vorformlinge (1) ist und gleich der oder höher als die Aktivierungstemperatur eines Desinfektionsmittellösungsaerosols ist;
eine Formungseinheit (4); wobei die Formungseinheit (4) zum Blasen der Vorformlinge (1) verwendet wird, um sterile Flaschen (5) herzustellen;
wobei die Sprüheinheit (2) ferner einen Ultraschallzerstäuber (6) umfasst, um eine Desinfektionsmittellösung in ein Desinfektionsmittellösungsaerosol umzuwandeln, und der Sprüher (7) zum Sprühen des Desinfektionsmittellösungsaerosols auf die Vorformlinge (1) dient, um eine im Wesentlichen gleichmäßige dünne Desinfektionsmittellösungsschicht auf mindestens die Innenflächen der zu desinfizierenden Vorformlinge (1) aufzubringen;
die Heizeinheit (3) dazu dient, die Vorformlinge (1) zu erhitzen, nachdem sie von der Sprüheinheit (2) besprüht wurden, um mindestens die Innenflächen der Vorformlinge (1) zu desinfizieren;
die Formungseinheit (4) dazu dient, die Vorformlinge (1) mit der von der Heizeinheit (3) erhitzten Blasformtemperatur zu blasformen, um sterile Flaschen (5) herzustellen;
die Sprüheinheit (2) ferner eine Heizung zum Erhitzen der Desinfektionsmittellösung oder des Desinfektionsmittellösungsaerosols umfasst, um eine Temperatur des Desinfektionsmittellösungsaerosols auf eine zweite Temperatur zu erhöhen, die höher als die Temperatur der Vorformlinge (1) vor dem Erhitzen in der Heizungseinheit (3) und niedriger als die erste Temperatur ist, und der Sprüher (7) dazu dient, das Desinfektionsmittellösungsaerosol mit der zweiten Temperatur auf die Vorformlinge (1) zu sprühen, um eine im Wesentlichen gleichmäßige dünne Desinfektionsmittellösungsschicht auf mindestens die Innenflächen der zu desinfizierenden Vorformlinge (1) aufzubringen.

5. Flaschenherstellungsvorrichtung nach Anspruch 4, **dadurch gekennzeichnet, dass** die Heizeinheit (3) dazu dient, die Vorformlinge (1) nach dem Besprühen durch die Sprüheinheit (2) auf die erste Temperatur zu erhitzen, die gleich der oder höher als die Verdampfungstemperatur des Desinfektionsmittellösungsaerosols ist, um die Desinfektionsmittellösung aus den Vorformlingen (1) zu verdampfen.

6. Flaschenherstellungsvorrichtung nach Anspruch 4, **dadurch gekennzeichnet, dass** die Desinfektionsmittellösung eine wasserstoffperoxidhaltige Lösung ist, die nach dem Erhitzen der Vorformlinge (1) auf eine die Aktivierungstemperatur übersteigende Temperatur zu aktivieren ist.

## Revendications

1. Procédé de désinfection de préformes, les préformes (1) étant désinfectées et moulées dans un dispositif de production de bouteilles stériles (5), le procédé de désinfection comprenant au moins l'étape suivante :
(1) transformer une solution désinfectante en aérosol de solution désinfectante au moyen d'un atomiseur à ultrasons (6), et pulvériser l'aérosol de solution désinfectante sur les préformes (1), de manière à déposer une couche mince sensiblement uniforme de solution désinfectante sur au moins les surfaces internes des préformes (1) à désinfecter ;
(2) chauffer les préformes (1) traitées à l'étape (1) par rayonnement pour élever la température d'au moins les surfaces internes des préformes (1) à une première température, où la première température est supérieure ou égale à la température d'activation de la solution désinfectante, de manière à désinfecter au moins les surfaces internes des préformes (1) ;
à l'étape (1), chauffer d'abord la solution désinfectante ou l'aérosol de solution désinfectante pour élever la température de l'aérosol de solution désinfectante à une deuxième température, puis pulvériser l'aérosol de solution désinfectante porté à la deuxième température sur les préformes (1), la deuxième température étant supérieure à la température des préformes (1) avant le chauffage à l'étape (2) et inférieure à la première température.

2. La méthode de désinfection des préformes selon la revendication 1, est **caractérisée en ce que** la méthode de désinfection comprend en outre une étape de désinfection auxiliaire :
(3) après l'étape (2), pulvériser la solution désinfectante en aérosol sur les préformes (1) chauffées à la première température à nouveau.

3. La méthode de désinfection des préformes selon la revendication 1, est **caractérisée en ce que** la première température est inférieure ou égale à la température de moulage par soufflage des préformes (1) et supérieure ou égale à la température de vaporisation de la solution désinfectante, de sorte que la solution désinfectante s'évapore des préformes (1).

4. Un dispositif de fabrication de bouteilles pour produire des bouteilles stériles (5) à partir de préformes en plastique (1), les préformes (1) étant transportées en continu vers le dispositif de fabrication de bouteilles, comprend les composants suivants disposés successivement dans le sens de transport des préformes (1) :
une unité de pulvérisation (2) ; l'unité de pulvérisation (2) comprend un pulvérisateur (7) pour pulvériser un milieu désinfectant au moins sur les surfaces internes des préformes (1) ;
une unité de chauffage (3) ; l'unité de chauffage (3) comprend un four de chauffage pour chauffer par rayonnement les préformes (1) à une première température qui est inférieure ou égale à la température de moulage par soufflage des préformes (1) et qui est supérieure ou égale à la température d'activation d'un aérosol de solution désinfectante ;
une unité de moulage ( 4) ; l'unité de moulage ( 4) est utilisée pour souffler les préformes (1) afin de fabriquer des bouteilles stériles ( 5) ;
dans lequel
l'unité de pulvérisation (2) comprend en outre un atomiseur à ultrasons ( 6) pour transformer une solution désinfectante en aérosol de solution désinfectante, et le pulvérisateur (7) est destiné à pulvériser l'aérosol de solution désinfectante sur les préformes (1) afin de fixer une couche mince sensiblement uniforme de solution désinfectante sur au moins les surfaces intérieures des préformes (1) à désinfecter ;
l'unité de chauffage (3) est destinée à chauffer les préformes (1) après qu'elles ont été aspergées par l'unité de pulvérisation (2), de manière à désinfecter au moins les surfaces internes des préformes (1) ;
l'unité de moulage (4) est destinée à mouler par soufflage les préformes (1) dont la température de moulage par soufflage est chauffée par l'unité de chauffage (3) pour fabriquer des bouteilles stériles (5) ;
l'unité de pulvérisation (2) comprend en outre un dispositif de chauffage pour chauffer la solution désinfectante ou l'aérosol de solution désinfectante afin d'élever la température de l'aérosol de solution désinfectante à une seconde température qui est supérieure à la température des préformes (1) avant chauffage dans l'unité de chauffage (3) et inférieure à la première température, et le pulvérisateur (7) est destiné à pulvériser l'aérosol de solution désinfectante à la seconde température sur les préformes (1), afin de fixer une couche mince sensiblement uniforme de solution désinfectante sur au moins les surfaces intérieures des préformes (1) à désinfecter.

5. Le dispositif de fabrication de bouteilles selon la revendication 4, est **caractérisé en ce que** l'unité de chauffage (3) est destinée à chauffer les préformes (1) après qu'elles ont été pulvérisées par l'unité de pulvérisation (2) à la première température qui est supérieure ou égale à la température de vaporisation de l'aérosol de solution désinfectante, de manière à évaporer la solution désinfectante des préformes (1).

6. Le dispositif de fabrication de bouteilles selon la revendication 4, est **caractérisé en ce que** la solution désinfectante est une solution contenant du peroxyde d'hydrogène, qui doit être activée après que les préformes (1) ont été chauffées à une température supérieure à la température d'activation.
